# EUROPÄISCHE PATENTSCHRIFT

(11) **EP 0 573 849 B1**
(45) Veröffentlichungstag und Bekanntmachung des Hinweises auf die Patenterteilung: **09.02.2000**
(21) Anmeldenummer: 93108560.9
(22) Anmeldetag: 27.05.1993
(51) Int. Cl.: C12N 9/18, C12P 35/00

(54) **7-Amino-3-methoxymethyl-3-cephem-4-carbonsäureester-Hydrolase, Verfahren zu deren Herstellung sowie deren Verwendung**
7-amino-3-methoxymethyl-3-cephem-4-carboxy acid ester-hydrolase, method of its production and its utilization
Hydrolase de 7-amino-3-méthoxyméthyl-3-cephem-4-ester d'acide carbonique, méthode pour sa fabrication et son utilisation

(30) Priorität: 06.06.1992 DE 4218785
(43) Veröffentlichungstag der Anmeldung: 15.12.1993
(73) Patentinhaber: HOECHST AKTIENGESELLSCHAFT, 65926 Frankfurt am Main (DE)
(72) Erfinder: Aretz, Werner, Dr., W-6240 Königstein/Ts. (DE); Sauber, Klaus, Dr., W-6232 Bad Soden/Ts. (DE)

(56) Entgegenhaltungen:
- JOURNAL OR ORGANIC CHEMISTRY, Bd.56, 1991, WASHINGTON DC US Seiten 3633 - 3637 SIGERU TORII ET AL. 'Deprotection of carboxylic esters ...'
- ENZYME AND MICROBIAL TECHNOLOGY, Bd.4, Nr.2, 1982, HAYWARDS HEATH GB Seiten 80 - 84 BERRY D.R ET AL. 'Enzymatic removal of a ...'

## Beschreibung

Bei der Synthese von oralen Cephalosporinen ausgehend von 7-Amino-3-methoxymethyl-3-cephem-4-carbonsäure (MACA) (I) fällt ein Isomeren-Gemisch von 7-Amino-3-methoxymethyl-3-cephem-4-carbonsäure-[1(R,S)-1-(2.2-dimethylpropionyloxy)-ethylester] (Pro-J- bzw. Pro-K-MACA-Ester) (II) an.

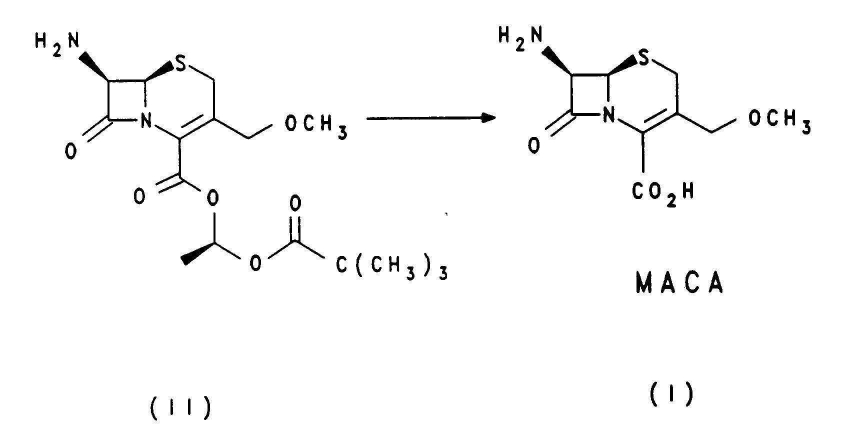

Nach Trennung in R- und S-Diastereomer muß das unerwünschte R-Diastereomer, das noch S-Anteil enthält durch eine nicht stereo- aber chemoselektive Reaktion in (I) zurückgeführt werden.

Die literaturbekannten Methoden der Esterspaltung erfordern Reaktionsbedingungen, die eine Anwendung auf 7-Amino-3-methoxymethyl-3-cephem-4-carbonsäureester wegen des labilen β-Lactamringes nicht in befriedigender Weise zulassen. So führt z.B. die alkalische Hydrolyse von Estern mit Basen wie KOH, NaOH oder Natriumalkoholaten in Wasser oder in organischen Lösungsmitteln wie z.B. Dioxan oder Alkohol lediglich zu Zersetzungsprodukten. Von S. Torii et al. ist die Spaltung von Cephalosporinestern mittels Phenol und Säurekatalyse beschrieben (J. Org. Chem. 56 (1991) 3633). Eine Anwendung dieses Verfahrens auf 7-Amino-3-methoxymethyl-3-cephem-4-carbonsäureester lieferte jedoch die gewünschte Carbonsäure lediglich in geringer Ausbeute und ungenügender Reinheit.

Auch die gut zugängliche Schweineleberesterase ist für die genannte Reaktion nicht geeignet, da sie ausschließlich zur Spaltung des S-Diastereomers führt.

Es wurde nun überraschenderweise gefunden, daß eine mikrobiologisch gewonnene Esterase geeignet ist, die oben beschriebene Esterspaltung chemoselektiv in guter Ausbeute zu bewirken. Das Enzym, kurz: MACA-Ester-Hydrolase genannt, wird u.a. von Pseudomonaden und Actinomyceten insbesondere der Gattungen Streptomyces und Amycolatopsis gebildet und in die Kulturbrühe abgegeben.

Erfindungsgegenstand ist demzufolge eine Esterase, die dadurch gekennzeichnet ist, daß sie die oben beschriebene Umwandlung der Verbindung der Formel II in die Verbindung der Formel I bewirken kann und herstellbar ist durch Kultivierung der Mikroorganismen Streptomyces bambergiensis oder Amycolatopsis orientalis.

Ganz besonders geeignete Stamme sind Streptomyces bambergiensis ATCC 13879, Amycolatopsis orientalis ATCC 14930 und Amycolatopsis orientalis ATCC 53492.

Das Verfahren zur Herstellung der genannten Esterase, das ebenfalls Gegenstand der vorliegenden Erfindung ist, ist wie folgt charakterisiert:
Die Kultivierung der betreffenden Mikroorganismen erfolgt unter üblichen Bedingungen. Vorzugsweise erfolgen Anzucht und Kultivierung auf Komplexmedien mit z.B. Cornsteep, Fleischextrakt, Pepton, Casein, Hefeextrakt, Gelatine, Trypton, Nitrat oder Ammonium als Stickstoffquellen und löslicher Stärke, Dextrin, Saccharose, Glucose oder Glycerin als Kohlenstoffquellen. Als Mineralien können u.a. Magnesium, Eisen, Calcium, Natrium, und Kobalt zugesetzt werden.

Die Kultivierung erfolgt vorzugsweise bei Raumtemperatur oder leicht darüber, insbesondere bei ca. 28°C. Die Kultivierungsdauer beträgt z. B. 48-60 Std. Die gegebenenfalls nötige Isolierung der Esterase erfolgt auf übliche Weise, z.B. durch Abfiltrieren oder Abzentrifugieren, wobei die Esterase sich im Überstand befindet, der nachfolgend z.B. lyophilisiert werden kann oder einer Ultrafiltration unterzogen werden kann. Weitere Reinigungsschritte können angezeigt sein und z.B. eine Fällstufe (z.B. mit Ammoniumsulfat) und/oder eine weitere Zentrifugation beinhalten. Für den Einsatz der Esterase kann es auch sinnvoll sein, dieselbe auf einem geeigneten Träger zu immobilisieren.

Für die Immobilisierung von gereinigten, teilweise gereinigten oder rohen Zellextrakten, die die Esterase enthalten, kommen z. B. trägergebundene Immobilisierungsverfahren in Frage. Beispielsweise kann die Esterase durch eine covalente Bindung über einen zur Katalyse nicht essentiellen Lysinrest an den polymeren Träger gekuppelt werden. Eine weitere Möglichkeit ist, die Adsorption der Esterase an einen Träger sowie die anschließende Quervernetzung mit z. B. Glutardialdehyd.

Als Enzymträger kommen z. B. polymere, poröse Träger wie Cellulosen, z. B. DEAE- oder CM-Cellulosen, Sepharosen, wie z. B. BrCN- oder Divinylsulfonaktivierte Sepharosen, modifizierte Polyacrylamidgele mit Amino- oder Hydroxylgruppen oder verschiedene organische Copolymerisate aus Acrylamid, Methacrylaten oder Methacrylamid und Maleinsäureanhydrid in Frage. Ferner können als Enzymträger auch Copolymere aus Glycidylmethacrylat, Allylglycidylether, Methylenbismethylacrylamid und Methacrylamid, wie z. B. ®Eupergit, eingesetzt werden.

Weitere geeignete Enzymträger sind vernetzte Polymere auf Basis von Polyvinylestern und Polyvinylalkoholen nach der DE-OS 33 44 912. Die Verankerungsreaktion der Esterase auf dem Enzymträger erfolgt in bekannter Weise, wie etwa in der DE-OS 2 215 687 beschrieben. Ein besonders geeigneter Enzymträger ist VA-Epoxy-Biosynth der Firma Riedel de Haen.

Ein Enzym aus A. orientalis hat ein Molekulargewicht von 55 200 ± 300 Da (Natriumdodecylsulfat-Polyacrylamid Gelelektrophorese) und eine spezifische Aktivität von ca. 15 mU/mg im Kulturfiltrat. Das pH-Optimum liegt bei 6.5. bis 7.5.

Es weist bei Raumtemperatur und pH 7.0 eine für technische Zwecke ausreichende Stabilität von mehreren Tagen auf. Das Enzym läßt sich nach Teilreinigung problemlos z.B. an den Enzymträger VA-Epoxy-Biosynth^{R} binden. Die Aktivität wird normalerweise durch Umsetzung von MACA-Ester zu MACA bestimmt. Dabei wird die HPLC oder DC zur quantitativen Auswertung der gebildeten MACA-Menge herangezogen.

Methoden zur Bestimmung der enzymatischen Aktivität:
DC-System: HPTLC Kieselgel 60 F₂₅₄ 10*20
1-Butanol: Eisessig:Ethanol:H₂O 50:20:15:15
®Desaga Densitometer CD 50
HPLC-System:
HPLC - Analytik:
Säule: ®LiChrospher 100 RP 18, 5 µm
Mob. Phase: A = 0,1% Ammoniumacetat + Tetrabutylammoniumhydrogensulfat(10mg/l) B = A + 80% Acetonitril
Gradient:

| t (min) | Fluß (ml/min) | %B |
|---|---|---|
| 0 | 1,0 | 4 |
| 6 | " | 8 |
| 10 | " | 70 |
| 18 | " | 80 |
| 20 | " | 4 |
| 24 | " | 4 |

Proben-Vol.: 10 bzw. 20 µl
Wellenlänge : 260 nm

Die erfindungsgemäße Esterase eignet sich zur Spaltung verschiedener Carbonsäureester. Besonders geeignet ist sie zur Spaltung von Estern von Mono- oder Dicarbonsäuren mit bis zu 6 C-Atomen, die ggf. mit F, Cl oder Br substituiert sein können, von natürlich vorkommenden Aminosäuren oder von der 7-Amino-3-methoxymethyl-3-cephem-4-carbonsäure
mit einem ein-, zwei- oder 3-wertigen Alkohol mit bis zu 6 C-Atomen, in dem die Kohlenstoffkette auch durch eine -C(O)O-Gruppe unterbrochen sein kann, oder mit Phenol, das auch durch Nitro substituiert sein kann.

Besonders geeignet ist die Esterase zur Spaltung von p-Nitrophenylacetat, 7-Amino-3-methoxymethyl-3-cephem-4-carbonsäure-[1(R,S)-1-(2.2-dimethylpropionyloxy)-ethylester], 7-Amino-3-methoxymethyl-3-cephem-4-carbonsäure-[(2.2-dimethylpropionyloxy)-methylester], 1,1-Bis(pivaloyloxy)-ethan, Propionsäureethylester, Pivalinsäureethylester, Triacetin, Tributyrin, Alaninethylester und 2-Chlorpropionsäureethylester.
Ganz besonders geeignet ist das Verfahren zur Spaltung von Amino-3-methoxymethyl-3-cephem-4-carbonsäure-[1(R,S)-1-(2.2-dimethylpropionyloxy)-ethylester], Propionsäureethylester, Pivalinsäureethylester, Alaninethylester und 2-Chlorpropionsäureethylester, insbesondere zur Spaltung von 7-Amino-3-methoxymethyl-3-cephem-4-carbonsäure-[1(R,S)-1-(2.2-dimethylpropionyloxy)-ethylester].

Die Spaltung der Ester, stellvertretend für die Umsetzung der Verbindung der Formel II zur Verbindung der Formel I erläutert, findet vorzugsweise unter folgenden Bedingungen statt.
Die Verbindung der Formel II wird in wäßriger, vorzugsweise auf im Bereich pH 6.8 gepufferter Lösung (z.B. Kaliumphosphatpuffer) mit der Esterase behandelt, bevorzugt mit der Esterase in geeignet fixierter Form. Vorzugsweise läßt man die Reaktion bei 25-35°C, insbesondere bei ca 30°C ablaufen. Nach ca. 6-8 Std. ist in der Regel die Reaktion zu 95% abgeschlossen. Die Reaktionslösung wird nach bekannten Methoden, z.B. Filtration, vom Enzym getrennt. Die Verbindung der Formel I wird isoliert, z.B. durch Lyophilisation, und anschließend ggf. weiter gereinigt, z.B. durch Umkristallisation.
Im folgenden soll die Erfindung durch Beispiele und den Inhalt der Patentansprüche näher erläutert werden.

### Beispiel 1

Die Überprüfung geeigneter Hydrolaseproduzenten erfolgte nach Vor- und Hauptanzucht der Stämme in Müller Hinton Medium (Difco) bei 28°C und 240 Upm.
Nach 3 Tagen wurden folgende Hydrolase-Aktivitäten ermittelt:

| Stamm | | %MACA-Bildung nach 16 Std. |
|---|---|---|
| Str. bambergiensis | ATCC 13879 | 6 |
| Amycolatopsis orientalis | ATCC 14930 | 18 |
| Amycolatopsis orientalis | ATCC 53492 | 39 |

### Beispiel 2

Die Vorkultur von A. orientalis ATCC 53492 wird im Medium folgender Zusammensetzung durchgeführt:

| | |
|---|---|
| Glucose | 20g/L |
| Hefeextrakt | 24g/L |
| Sojamehl | 8g/L |
| NaCl | 1g/L |
| CaCO₃ | 4g/L |
| pH 7,0 | |

500ml/2000ml Erienmeyerkolben; 28°C; 240 Upm; 3 Tage

### Hauptkulturmedium:

| | |
|---|---|
| lösl.Stärke | 10g/L |
| Glucose | 10g/L |
| Glycerin | 10g/L |
| Pepton | 5g/L |
| Cornsteep fl. | 2,5g/L |
| Hefeextrakt | 2g/L |
| NaCl | 1g/L |
| pH 7,2 | |

9L Medium/12L Fermenter; Inokulum=6%; 28°C; 350Upm; Belüftungsrate: 0,5vvm

Nach 48-60 Stunden wird die maximale Hydrolase-Aktivität von >90% MACA-Bildung in 4 Std. erreicht.

### Beispiel 3

3.5 l Kulturlösung aus Beispiel 2 werden durch Zentrifugation in Kulturüberstand und Biomasse getrennt. Das Enzym befindet sich im Überstand. Diese Lösung wird lyophilisiert. 85g Feststoff werden in 500ml 50mM Kaliumphosphatlösung, pH =7.8, eingerührt und anschließend zentrifugiert. Die fast klare, dunkelbraune Lösung wird einer Ammoniumsulfatfällung unterworfen. Statt der Lyophilisation kann das Kulturfiltrat auch mittels Ultrafiltration (cut-off: 10 000 Da) auf 500ml konzentriert werden. Dann wird Ammoniumsulfat ad 35% der Sättigung zugegeben und abermals zentrifugiert. Der Überstand wird auf 70% der Sättigung mit Ammoniumsulfat aufgestockt. Nach einer weiteren Zentrifugation bei mind. 13000g wird der Niederschlag in 250ml Puffer (s.o.) aufgenommen. Die Aktivität dieser Lösung beträgt 370mU/ml.

### Beispiel 4

Zu 250ml Enzymlösung nach Beispiel 3 wird soviel prim. und sek. Phosphat gegeben, daß die Lösung 1 M an Phosphat ist. Der pH beträgt 8.0. Eine leichte Trübung wird in Kauf genommen. Dann werden 50g Enzymträger VA-Epoxy Biosynth^{R} (Riedel de Haen, Seelze, Deutschland) hinzugegeben und 3 Tage bei Raumtemperatur stehengelassen. Dann wird mit 1 M NaCl und mit bidest. Wasser gewaschen. 72% der Enzymaktivität befinden sich auf dem Träger, der eine spez. Aktivität von 1.35 U/g TGW aufweist.

### Beispiel 5

2.5g R-Ester-Hydrochlorid werden in 800ml 50mM Kaliumphosphatlösung, pH=6.8, gelöst. Dann werden unter Rühren 100g feuchtes fixiertes Enzym zugegeben. Der pH wird bei 6.8 und die Temperatur auf 30°C gehalten. Nach 2.5 h werden weitere 1.25 und nach 4 1/4 h nochmals 1.25g Substrat zugegeben. Insgesamt enthält der Ansatz 5g MACA-Ester (R). Die Spaltung verläuft innerhalb von 6-8 Stunden bis auf 95% Umsatz laut HPLC. Die vom Enzym getrennte Spaltlösung wird lyophilisiert. Man erhält ca. 10g eines weißen Feststoffes.

### Beispiel 6

Der aus Beispiel 5 gewonnene Feststoff wird in 35ml Wasser aufgenommen, pH=6.8 eingestellt (verd. Ammoniak) und ein unlöslicher Rückstand abgetrennt.
Dann wird die klare, gelbliche Lösung auf 10° abgekühlt und unter Rühren 5 N HCl zugegeben. Ab pH=5.5 beginnt die MACA auszukristallisieren. Der pH wird weiter auf 2.5 abgesenkt und 20 Minuten nachgerührt. Die Kristalle werden über Seitz-Filter abgetrennt. Es wird dann mit Wasser, Aceton und schließlich Diisopropyläther nachgewaschen. Der Feststoff wird im Vakuum bei R.T. getrocknet.

Auswaage: 2.2g (96%-ig) entsprechend 71% Ausbeute, bezogen auf eingesetzten MACA-Ester. 0.34g MACA, entsprechend 11.4%, verbleiben in der Mutterlauge.

Die folgende Tabelle enthält weitere Informationen über die Substratspezifität der erfindungsgemäßen Esterase:

| Substrat | Konzentration | Aktivität | | % |
|---|---|---|---|---|
| | | U/ml | U/mg | |
| p-Nitrophenylacetat (HEPES, pH 6.95) | 1.59 mM | 30.4 | 76.0 | 86.4 |
| p-Nitrophenylacetat (Phosphatpuffer, pH 6,8) | 2.5 mM | 43.5 | 108.75 | 123.6 |
| MACA-Ester Pro J | 2.5 mM | 35.2 | 87.75 | 100 |
| MACA-Ester Pro K | 2.5 mM | 35.1 | 87.75 | 99.7 |
| 1,3-Bis(pivaloyloxy)-ethan | 50 mM | 1.4 | 3.5 | 4 |
| Propionsäureethylester | 100 mM | 20 | 50 | 56.8 |
| Pivalinsäureethylester | 100 mM | 17.1 | 42.75 | 48.6 |
| Triacetin | 100 mM | 37.1 | 92.75 | 105.4 |
| Tributyrin | 100 mM | 37.5 | 93.75 | 106.5 |
| D,L-Alaninethylester | 100 mM | 16 | 40 | 45.5 |
| 2-Chlorpropionsäureethylester | 100 mM | 30 | 75 | 85.2 |

## Patentansprüche

1. Esterase, dadurch gekennzeichnet, daß sie die chemoselektive Umwandlung der Verbindung der Formel II in die Verbindung der Formel I bewirken kann 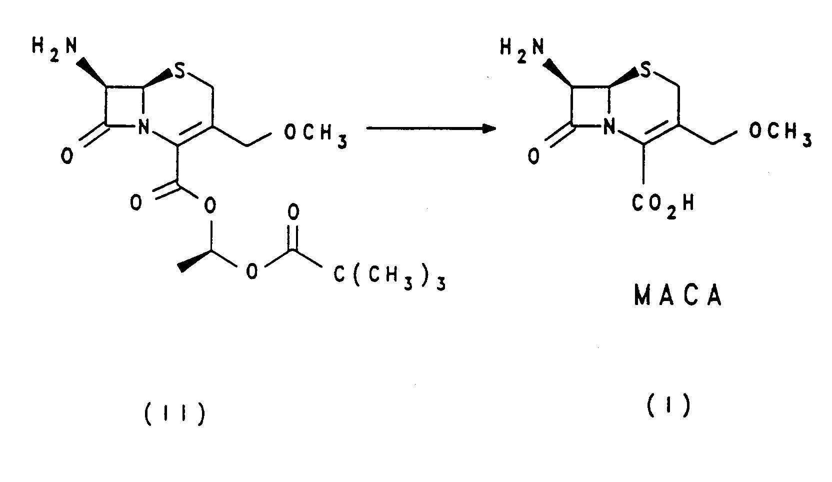 und herstellbar ist durch Kultivierung der Mikroorganismen Streptomyces bambergiensis oder Amycolatopsis orientalis.

2. Esterasegemäß Anspruch 1, dadurch gekennzeichnet, daß sie ein Molekulargewicht von ca. 55 200 ± 300 Da aufweist.

3. Verfahren zur Herstellung einer Esterase gemaß einem oder mehreren der Ansprüche 1-2, dadurch gekennzeichnet, daß ein geeigneter Mikroorganismus unter üblichen Bedingungen kultiviert wird und die Esterase ggf. aus der Kulturbrühe isoliert und ggf. gereinigt wird.

4. Verwendung einer Esterase gemäß einem oder mehreren der Ansprüche 1-2 zu chemoselektiver Spaltung von Estern von Mono- oder Dicarbonsäuren mit bis zu 6 C-Atomen, die ggf. mit F, Cl oder Br substituiert sein können, von natürlich vorkommenden Aminosäuren oder von der 7-Amino-3-methoxymethyl-3-cephem-4-carbonsäure
mit einem ein-, zwei- oder 3-wertigen Alkohol mit bis zu 6 C-Atomen, in dem die Kohlenstoffkette auch durch eine -C(O)O-Gruppe unterbrochen sein kann, oder mit Phenol, das auch durch Nitro substituiert sein kann.

5. Verwendung einer Esterase gemäß einem oder mehreren der Ansprüche 1-2 zur chemoselektiven Umwandlung der Verbindung der Formel II in die Verbindung der Formel I.

6. Verwendung einer Esterase gemäß den Ansprüchen 4 oder 5, dadurch gekennzeichnet, daß die Esterase auf einem Träger immobilisiert ist.

7. Verfahren zur Umwandlung einer Verbindung der Formel II in eine Verbindung der Formel I, dadurch gekennzeichnet, daß eine Verbindung der Formel II mit einer Esterase gemäß einem oder mehreren der Ansprüche 1-2 behandelt wird.

## Claims

1. An esterase which can bring about the chemoselective conversion of the compound of the formula II into the compound of the formula I 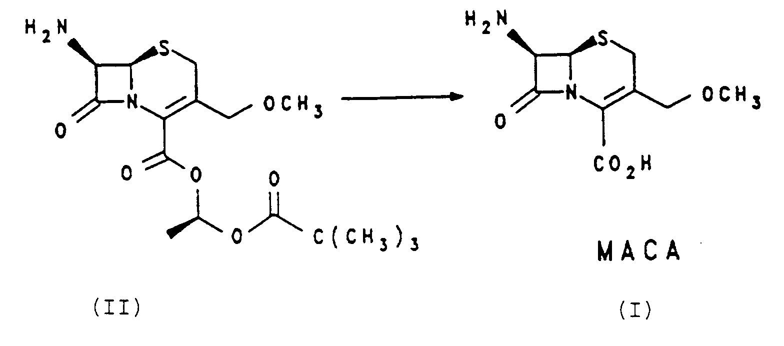 and which can be prepared by cultivation of the microorganisms Streptomyces bambergiensis or Amycolatopsis orientalis.

2. The esterase as claimed in claim 1, which has a molecular weight of about 55 200 ± 300 Da.

3. A process for preparing an esterase as claimed in one or more of claims 1-2, wherein a suitable microorganism is cultured under usual conditions and the esterase is optionally isolated from the culture broth and optionally purified.

4. The use of an esterase as claimed in one or more of claims 1-2 for chemoselective cleavage of esters of monocarboxylic acids or dicarboxylic acids having up to 6 carbon atoms, which may optionally be substituted by F, Cl or Br, of naturally occurring amino acids, or of 7-amino-3-methoxymethyl-3-cephem-4-carboxylic acid, with a monohydric, dihydric or trihydric alcohol having up to 6 carbon atoms, in which the carbon chain may also be interrupted by a -C(O)O- group, or with phenol, which may also be substituted by nitro.

5. The use or an esterase as claimed in one or more of claims 1-2 for the chemoselective conversion of the compound of the formula II into the compound of the formula I.

6. The use of an esterase as claimed in claims 4 or 5, wherein the esterase is immobilized on a carrier.

7. A process for converting a compound of the formula II into a compound of the formula I, wherein a compound of the formula II is treated with an esterase as claimed in one or more of claims 1-2.

## Revendications

1. Estérase, caractérisée en ce qu'elle peut provoquer la conversion chimiosélective du composé de formule II en le composé de formule I 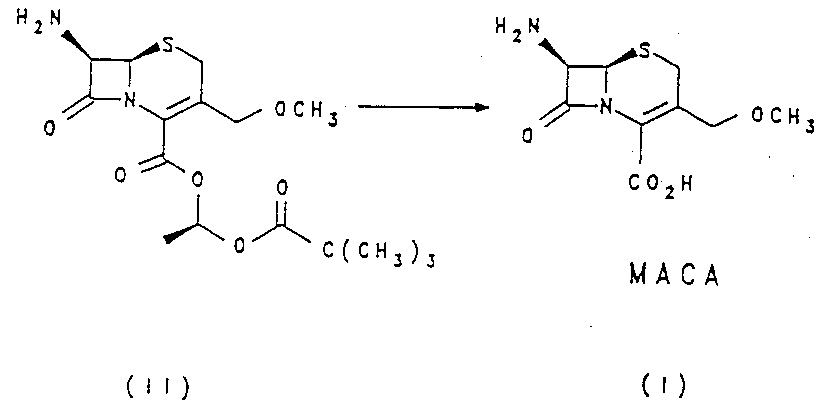 et peut être produite par culture du micro-organisme *Streptomyces banbergiensis* ou *Amycolatopsis orientalis*.

2. Estérase selon la revendication 1, caractérisée en ce qu'elle présente une masse moléculaire d'environ 55 200 ± 300 Da.

3. Procédé pour la production d'une estérase selon une ou plusieurs des revendications 1-2, caractérisé en ce qu'un micro-organisme approprié est cultivé dans les conditions usuelles, et l'estérase est éventuellement isolée à partir du bouillon de culture et éventuellement purifiée.

4. Utilisation d'une estérase selon une ou plusieurs des revendications 1-2, pour la coupure chimio-sélective d'esters d'acides mono- ou dicarboxyliques ayant jusqu'à 6 atomes de carbone, qui peuvent éventuellement être substitués par F, Cl ou Br, d'aminoacides existant dans la nature ou de l'acide 7-amino-3-méthoxyméthyl-3-céphème-4-carboxylique, avec un alcool mono-, di- ou tri-hydroxylé ayant jusqu'à 6 atomes de carbone, dans lequel la chaîne carbonée peut également être interrompue par un groupe -C(O)O-, ou avec un phénol qui peut également être substitué par le groupe nitro.

5. Utilisation d'une estérase selon une ou plusieurs des revendications 1-2, pour la conversion chimiosélective du composé de formule II en le composé de formule I.

6. Utilisation d'une estérase selon la revendication 4 ou 5, caractérisée en ce que l'estérase est immobilisée sur un support.

7. Procédé pour la conversion d'un composé de formule II en un composé de formule I, caractérisé en ce que l'on traite un composé de formule II par une estérase selon une ou plusieurs des revendications 1-2.
